Europäisches Patentamt

European Patent Office

Office européen des brevets

(19)

(11) Veröffentlichungsnummer: **0 001 122**
**B1**

(12) ## EUROPÄISCHE PATENTSCHRIFT

(21) Anmeldenummer: **78100855.2**

(22) Anmeldetag: **08.09.78**

(51) Int. Cl.³: **C 12 P 7/18, C 07 C 31/18**

(54) Verfahren zur Herstellung eines Zuckeralkohols.

(30) Priorität: **12.09.77 LU 78114**

(43) Veröffentlichungstag der Anmeldung:
**21.03.79 Patentblatt 79/6**

(45) Bekanntmachung des Hinweises auf die Patenterteilung:
**25.02.81 Patentblatt 81/8**

(84) Benannte Vertragsstaaten:
**CH DE FR GB**

(56) Entgegenhaltungen:
**DE - A - 1 926 178**

**CHEMICAL ABSTRACTS, vol. 59, 1963, 3288f**

**CHEMICAL ABSTRACTS, vol. 69, 1968, 95094V
T. HARADA et al: "Utilization of alcohols by
Hansenula miso"**

**CHEMICAL ABSTRACTS, vol. 82, 1975, 56007f
K. HATTORI et al: "Microbiol production of D-
arabitol by n-alkanegrown Candida tropicals"**

(73) Patentinhaber: **F. HOFFMANN-LA ROCHE & CO.
Aktiengesellschaft
CH-4002 Basel (CH)**

(72) Erfinder: **Fujiwara, Akiko
1059-7 Fueta
Kamakura-shi Kanagawa-ken (JP)**
(72) Erfinder: **Masuda, Setsuko
5 Kuritaya Kanagawa-ku
Yokohama-shi Kanagawa-ken (JP)**

(74) Vertreter: **Wuesthoff, Franz, Dr.-Ing. et al,
Patentanwälte Wuesthoff -v. Pechmann-Behrens-
Goetz Schweigerstrasse 2
D-8000 München 90 (DE)**

Courier Press, Leamington Spa, England.

## 0 001 122

### Verfahren zur Herstellung eines Zuckeralkohols

Die vorliegende Erfindung betrifft ein Verfahren zur Herstellung eines Zuckeralkohols, nämlich D-Arabit. Das erfindungsgemässe Verfahren, welches für die industrielle Verwendung geeignet ist, ist ein Fermentationsverfahren, wobei eine bestimmte Hefe verwendet wird.

Bei bisher bekannten Fermentationsverfahren zur Herstellung von D-Arabit werden Hefen der Gattungen Candida, Saccharomyces, Hansenula, Debaryomyces, Torulopsis, Pichia oder Endomycopsis verwendet, wobei Nährmedien eingesetzt werden, welche Kohlenhydrate, wie Glucose oder Sucrose, oder Glycerin als die hauptsächlichen Kohlenstoffquellen enthalten. Diese bekannten Verfahren haben jedoch verschiedene Nachteile. Die als Nährmedien verwendeten Substanzen wie Glucose, Glycerin und Sucrose sind teuer. Ferner ist es sehr schwierig, den bei diesen Verfahren gebildeten D-Arabit von den als Kohlenstoffquelle verwendeten Zuckern zu trennen.

Es ist auch bereits ein Verfahren zur Herstellung- von D-Arabit durch Kultivieren einer Hefe der Gattung Candida oder Torulopsis bekannt, bei welchem wesentlich billigere Ausgangsmaterialien, wie Kohlenwasserstoffe oder Aethylalkohol als die Hauptkohlenstoffquelle verwendet werden (Agricultural and Biological Chemistry, Band 38, 1875—1888, 1974). In diesem Artikel wird berichtet, dass die Hefe Pichia membranfaciens in einem Kohlenwasserstoffe als die Hauptkohlenstoffquelle enthaltenden Nährmedium überhaupt kein D-Arabit erzeugt. Wie in eigenen Versuchen festgestellt wurde, liefern auch andere Mikroorganismen der Gattung Pichia, beispielsweise P. fluxnum, P. fermentans und P. toletana, in Kohlenwasserstoffe enthaltenden Kulturmedien kein D-Arabit.

Ueberraschenderweise wurde nun festgestellt, dass eine bestimmte Spezies von Pichia, nämlich Pichia haplophila und die davon abgeleiteten Mutanten, aus Kohlenwasserstoffen oder Aethylalkohol D-Arabit in hohen Ausbeuten produzieren.

Demgemäss besteht das erfindungsgemässe Verfahren zur Herstellung von D-Arabit darin, das man Mikroorganismen der Spezies Pichia haplophila oder Mutanten hiervon in einem Nährmedium kultiviert, dessen Kohlenstoffquelle im wesentlichen aus Kohlenwasserstoffen oder Aethylalkohol besteht.

Der so gewonnene Arabit kann in einfacher Weise aus der Kulturbrühe gewonnen werden.

Die beim erfindungsgemässen Verfahren verwendeten D-Arabit-produzierenden Mikroorganismen sind Hefen der Spezies Pichia haplophila, welche D-Arabit aus Kohlenwasserstoffen oder Aethylalkohol herstellen können, und Mutanten hiervon. Solche Mutanten kann man aus den Elternstämmen nach üblichen Mutierungsmethoden, beispielsweise durch Bestrahlung mit Ultraviolettstrahlung, Röntgenstrahlung oder Gamma-Strahlung oder durch Behandlung mit geeigneten Mutagenen erhalten.

Die nach dem erfindungsgemässen Verfahren bevorzugt verwendeten Stämme von Pichia haplophila sind bei NRRL und FRI hinterlegt. Es handelt sich hierbei um die Stämme Pichia haplophila NRRL 11,175 (FERM P-3955) und Pichia haplophila NRRL 11,176 (FERM P-3956). Der letztere Stamm wurde vom Stamm Pichia haplophila NRRL 11,175 (FERM P-3955) durch UV-Bestrahlung erhalten.

Bei Durchführung des erfindungsgemässen Verfahrens werden die D-Arabit-produzierenden Mikroorganismen in einem flüssigen Medium kultiviert, welches die oben angegebenen Kohlenstoffquellen, Stickstoffquellen, anorganische Salze und, falls erforderlich, andere organische oder anorganische Nährstoffe enthält. Als Kohlenwasserstoffe können flüssige oder feste Paraffine, Olefine und Isoparaffine mit verschiedener Kohlenstoffanzahl oder Gemische hiervon verwendet werden. Die alternative Kohlenstoffquelle ist Aethylalkohol. Die Konzentration der Kohlenstoffquelle hängt vom verwendeten Stamm und den Kulturbedingungen ab. Bevorzugte Konzentrationen liegen bei der Verwendung von Kohlenwasserstoffen im Bereich zwischen etwa 3 und etwa 30 Vol.% und bei Verwendung von Aethylalkohol zwischen etwa 1 und 20 Vol.%, jeweils bezogen auf das Gesamtvolumen des Kulturmediums.

Als Stickstoffquelle können verwendet werden Pepton, Maisquellwasser, Hefeextrakt, Fleischextrakt, Ammoniumsulfat, Ammoniumnitrat, Harnstoff und dergleichen, und zwar entweder allein oder in Kombination. Das Kulturmedium kann ferner anorganische Salz, wie beispielsweise Calciumcarbonat, Natriumchlorid, Natriumphosphat und dergleichen, sowie Zusätze wie Vitamine, Aminosäuren und dergleichen enthalten.

Die Kultivierung wird zweckmässig unter aeroben Bedingungen, insbesondere mittels Schüttelkultur oder Submers kultur durchgeführt. Die optimale Kultivierungstemperatur liegt im Bereich zwischen etwa 20 und etwa 40°C und der optimale pH zwischen etwa 2,0 und 8,0, vorzugsweise zwischen etwa 2,0 und 6,0. Die Fermentationszeit beträgt im allgemeinen zwischen etwa 3 und 10 Tagen.

Die Isolation des D-Arabit aus der Fermentationsbrühe kann unter Verwendung an sich bekannter Verfahren erfolgen, beispielsweise mittels Ionenaustauscharzen oder durch Lösungsmittelextraktion.

Der so erhaltene Polyalkohol D-Arabit ist als Süssstoff geeignet und findet auch Verwendung als Zwischenprodukt zur Herstellung anderer Verbindungen.

Die Anzahl der Kohlenstoffatome der oben genannten Paraffine, Isoparaffine und Olefine beträgt zweckmässig 10—20, vorzugsweise 13—16.

Die (z.B. im ersten Absatz von Seite 3) genannten beiden Mikroorganismen sind am *22. August 1978* bei *ATCC* unter den *Nummern 20518* und *20519* hinterlegt worden.

**0 001 122**

Ergänzt gemäss Antrag vom 2.11.1978, eingegangen am 3.11.1978.

## Beispiel 1

30 ml eines Nährmediums, welches 5% (Gew./Vol.) n-Paraffine Zusammensetzung: $C_{13}$ 0,3%, $C_{14}$ 50%, $C_{15}$ 49,5% und $C_{16}$ 0,2%), 0,2% (Gew./Vol) Ammoniumchlorid, 0,05% (Gew./Vol.) Monokaliumphosphat, 0,05% (Gew./Vol.) $MgSo_4.7H_2O$, 0,1% (Gew./Vol.) Hefeextrakt und 0,5% (Gew./Vol.) Calciumcarbonat in einem 500 ml-Kolben wird mit Pichia haplophila NRRL 11,176 (FERM P-3956) angeimpft. Dieser Stamm ist der von Pichia haplophila NRRL 11,175 (FERM P-3955) durch Bestrahlung der Zellsuspension mit einer UV-Lampe im Abstand von 30 cm, während 5 Minuten erhaltene Mutant. Die Fermentation erfolgt während 6 Tagen bei 30°C auf einer rotierenden Schüttelvorrichtung. Man erhält etwa 10 mg D-Arabit pro ml Kulturmedium. Nach Entfernung der Zellen durch Abzentrifugieren lässt man das Filtrat über ein Kationenaustauschharz (Dowex 50W x 4, (Warenzeichen, von Dow Chemical Co geliefert) $H^+$-Typ) und ein Anionenaustauschharz (Amberlite IR-45, (Warenzeichen, von Rohm und Haas Co geliefert) $OH^-$-Typ) laufen und dampft den Durchlauf unter vermindertem Druck zur Trockene ein. Der Rückstand wird mit warmem Aethylalkohol versetzt und der Extrakt auf ein kleineres Volumen eingedampft und bei 5°C über Nacht stehen gelassen. Man erhält 170 mg D-Arabit mit einem Schmelzpunkt von 102—103°C, $[\alpha]_D^{20}$ = +13,1 (c = 9,91 in gesättigter Boraxlösung).

## Beispiel 2

Es wird in zu Beispiel 1 analoger Weise vorgegangen, wobei jedoch anstelle von Pichia haplophila NRRL 11,175 der Stamm Pichia haplophila NRRL 11,175 (FERM P-3955) verwendet wird. Man erhält hierbei etwa 4 mg D-Arabit pro ml Nährmedium.

Wenn man den Versuch unternimmt, die Fermentation mit Pichia fluxuum IFO-0773, Pichia membranfaciens IFO-0864, Pichia membranfacience IFO-1004, Pichia fermentans NRRL y-1619 und Pichia toletana IFO-0950 durchzuführen, erhält man kein D-Arabit.

## Beispiel 3

In einem 500 ml-Kolben werden 30 ml eines Nährmediums (2% (Gew./Vol.) Aethylalkohol; 0,2% Ammoniumchlorid; 0,06% $KH_2PO_4$; 0,05% $MgSO_4.7H_2O$; 0,1% Hefeextrakt und 0,5% Calciumcarbonat) mit Pichia haplophila NRRL 11,175 (FERM P-3955) angeimpft und während 6 Tagen bei 30°C unter Schütteln fermentiert. Der Kultur werden zweimal je 6 ml Aethylalkohol zugesetzt, und zwar am 4. und am 5. Tag. Man erhält 3 mg D-Arabit pro ml Gärlösung.

Der in Beispiel 1 verwendete Stamm Pichia haplophila NRRL 11,176 (FERM P-3956) hat die folgenden Eigenschaften: Die Zellen sind rund bis oval und messen etwa 3,0 bis 5,1 x 2,0 bis 3,0 $\mu$m; sie treten einzeln, in Paaren oder in Gruppen auf. es bildet sich ein Sediment und ein dünnes, mattes, ausgebreitetes Häutchen. Die Stichkultur ist weiss und fein gefältelt. Es kann sich ein primitives Pseudomycel bilden. Auf Gipsblöcken wird eine Ascus-Bildung beobachtet. Die Sporen sind halbkreis-förmig.

Vergärung: negativ.

### Assimilierung von Kohlenstoffverbindungen:

| | | | |
|---|---|---|---|
| Glucose | + | D-Ribose | + |
| Galactose | + | L-Rhamnose | — |
| L-Sorbose | — | Aethanol | + |
| Sucrose | — | Glycerin | + |
| Maltose | — | Erythrit | + |
| Cellobiose | — | Ribit | + |
| Trehalose | — | Galactit | + |
| Lactose | — | D-Mannit | + |
| Melibiose | — | D-Glucit | + |
| Raffinose | — | $\alpha$-Methyl-D-glucosid | — |
| Melecitose | — | Salicin | — |
| Inulin | — | DL-Milchsäure | --- |

3

| Lösliche Stärke | w* | Bernsteinsäure | — |
| D-Xylose | + | Zitronensäure | — |
| L-Arabinose | + | Inosit | — |
| D-Arabinose | w | | |

*w = nur schwache Assimilierung beobachtbar

Spaltung von Arbutin: negativ
Assimilierung von Kaliumnitrat: negativ
Wachstum in einem vitaminfreien Medium: negativ
Wachstum auf einem 50%igen (Gew./w.) Glucose-Hefeextrakt-agar: negativ
Wachstum bei 37°C: negativ

Die oben angegebenen Eigenschaften stimmen überein mit denen des Elternstammes Pichia haplophila NRRL 11,175 (FERM P-3955) und auch mit der Beschreibung von Pichia haplophila in "The Yeasts: A taxanomic study" von N.J.W. Kreger-Van Rij, Seiten 492—494, 1970, North-Holland Publishing Company.

## Patentansprüche

1. Verfahren zur Herstellung von d-Arabit, dadurch gekennzeichnet, dass man Mikroorganismen der Spezies Pichia haplophila oder Mutanten hiervon in einem Nährmedium kultiviert, dessen Kohlenstoffquelle im wesentlichen aus Kohlenwasserstoffen oder Aethylalkohol besteht.

2. Verfahren nach Anspruch 1, dadurch gekennzeichnet, dass man als Mikroorganismus Pichia haplophila NRRL 11,175 (FERM P-3955) oder Pichia haplophila NRRL 11,176 (FERM P-3956) verwendet.

3. Verfahren nach Anspruch 1 oder 2, dadurch gekennzeichnet, dass man ein Nährmedium mit einem Kohlenwasserstoffgehalt von etwa 3% bis etwa 30% (Vol./Vol.) verwendet.

4. Verfahren nach Anspruch 1 oder 2, dadurch gekennzeichnet, dass man ein Nährmedium mit einem Aethylalkoholgehalt von etwa 1% bis etwa 20% (Vol./Vol.) verwendet.

5. Verfahren nach einem der Ansprüche 1—4, dadurch gekennzeichnet, dass man die Kultivierung bei Temperaturen zwischen etwa 20° und 40°C durchführt.

6. Verfahren nach einem der Ansprüche 1—5, dadurch gekennzeichnet, dass man die Kultivierung bei einem pH von etwa 2 bis etwa 8, vorzugsweise von etwa 2—6 durchführt.

## Revendications

1. Procédé d'obtention de D-arabitol caractérisé en ce qu'on cultive des microorganismes de l'espèce Pichia haplophila ou ses mutants dans un milieu nutritif dont la source de carbone est constituée essentiellement d'hydrocarbures ou d'alcool éthylique.

2. Procédé selon la revendication 1, caractérisé en ce qu'on utilise comme microorganisme Pichia haplophila NRRL 11,175 (FERM P-3955) ou Pichia haplophila NRRL 11,176 (FERM P-3956).

3. Procédé selon l'une des revendications 1 et 2, caractérisé en ce qu'on utilise un milieu nutritif ayant une teneur en hydrocarbures d'environ 3 à 30% en volume/volume.

4. Procédé selon l'une des revendications 1 et 2, caractérisé en ce qu'on utilise un milieu nutritif ayant une teneur en alcool éthylique d'environ 1 à 20% en volume/volume.

5. Procédé selon l'une des revendications 1 à 4, caractérisé en ce qu'on effectue la culture à des températures comprises entre 20 et 40°C environ.

6. Procédé selon l'une des revendications 1 à 5, caractérisé en ce qu'on effectue la culture à un pH compris entre 2 et 8 environ, de préférence entre 2 et 6.

## Claims

1. Process for the production of D-arabitol, characterized in that microorganisms of the species Pichia haplophila or mutants thereof are cultivated in a nutrient medium, the carbon source of which consists essentially of hydrocarbons or ethyl alcohol.

2. Process according to claim 1, characterized in that Pichia haplophila NRRL 11,175 (FERM P-

**0 001 122**

3955) or Pichia haplophila NRRL 11,176 (FERM P-3956) is used as microorganism.

3. Process according to claim 1 or 2, characterized in that a nutrient medium having a hydrocarbon content of about 3% to about 30% (vol./vol.) is used.

4. Process according to claim 1 or 2, characterized in that a nutrient medium having an ethyl alcohol content of about 1% to about 20% (vol./vol.) is used.

5. Process according to any one of claims 1 to 4, characterized in that the cultivation is carried out at temperatures between about 20° and 40°C.

6. Process according to any one of claims 1 to 5, characterized in that the cultivation is carried out at a pH of about 2 to about 8, preferably of about 2—6.